# FASCICULE DE BREVET EUROPEEN

(11) **EP 4 048 185 B1**
(45) Date de publication et mention de la délivrance du brevet: **16.07.2025**
(21) Numéro de dépôt: 20807467.4
(22) Date de dépôt: 20.10.2020
(51) Int. Cl.: A61B 34/30, A61B 17/00, A61N 1/05

(54) **INSTRUMENT ET INSTALLATION ROBOTISÉE DE CHIRURGIE OTOLOGIQUE POUR LA CAPTURE ET LE MAINTIEN D'UN PORTE-ÉLECTRODES D'IMPLANT COCHLÉAIRE**
INSTRUMENT UND ROBOTERANLAGE FÜR DIE OTOLOGISCHE CHIRURGIE ZUR AUFNAHME UND ZUM HALTEN EINES ELEKTRODENHALTERS EINES COCHLEAIMPLANTATS
INSTRUMENT AND ROBOTIC INSTALLATION FOR OTOLOGIC SURGERY FOR CAPTURING AND HOLDING A COCHLEAR IMPLANT ELECTRODE HOLDER

(30) Priorité: 22.10.2019 FR 1911806
(43) Date de publication de la demande: 31.08.2022
(73) Titulaire: Collin, 92220 Bagneux (FR)
(72) Inventeur: MAZALAIGUE, Stéphane, 92220 Bagneux (FR)
(74) Mandataire: Bonnet, Michel
(86) Numéro de dépôt international: PCT/FR2020/051893
(87) Numéro de publication internationale: WO 2021/079058

(56) Documents cités:
- WO-A1-00/71063
- WO-A1-2013/166293
- CN-A- 108 211 112
- FR-A1- 3 066 378
- US-A1- 2018 050 196

## Description

La présente invention concerne un instrument de chirurgie otologique pour l'insertion d'un porte-électrodes d'implant cochléaire dans une oreille interne d'un patient. Elle concerne également une installation robotisée comportant un tel instrument.

L'invention s'applique plus particulièrement à un instrument de chirurgie otologique de ce type, comportant une extrémité proximale de préhension et une extrémité distale fonctionnelle de capture et maintien d'une portion du porte-électrodes pendant l'insertion.

Un implant cochléaire est constitué d'une partie externe et d'une partie interne, toutes deux constituées de plusieurs éléments. La partie externe, destinée à être portée à l'oreille et contre la peau du crâne d'un individu, comporte généralement au moins un microphone qui capte un environnement sonore et transforme le son en signal électrique, un processeur qui filtre les informations reçues, notamment pour traiter en priorité la voix, et un transmetteur par induction électromagnétique. La partie interne comporte un récepteur placé sous la peau en vis-à-vis du transmetteur, relié électriquement à un porte-électrodes destiné à être placé dans la rampe tympanique de la cochlée de l'individu. Le porte-électrodes est un élément souple et élastique, généralement constitué de silicone qu'il est difficile d'insérer dans l'oreille interne lors de l'intervention chirurgicale nécessaire à l'installation de la partie interne de l'implant chez un patient.

Un exemple d'instrument de chirurgie otologique pour l'insertion d'un porte-électrodes d'implant cochléaire dans une oreille interne d'un patient est par exemple décrit dans la demande de brevet EP 3 513 834 A1. Il présente en partie distale une canule dans laquelle est installé le porte-électrodes avant intervention et un piston permettant de pousser le porte-électrodes pendant l'intervention pour son extraction de la canule et son insertion dans la cochlée du patient. Il présente également une portion proximale cylindrique de préhension pouvant être directement prise en main par un chirurgien. En variante, cette portion proximale cylindrique peut être prise dans le crochet d'un bras de robot bien qu'elle ne soit pas spécifiquement prévue pour cela. L'instrument lui-même s'avère assez complexe à fabriquer.

Un autre exemple d'installation robotisée pour l'insertion d'un porte-électrodes d'implant cochléaire est décrit dans le brevet US 8,594,799 B2. L'installation est illustrée dans ce document, mais l'instrument de capture et maintien du porte-électrodes n'est pas détaillé. Il est indiqué qu'un instrument habituel d'insertion peut être utilisé, sans indiquer lequel et sans réellement expliquer comment. Les documents US 2018/050196, FR 3 066 378 et WO 2013/166293 décrivent d'autres systèmes connus.

Un exemple d'instrument habituel est une micro-pince articulée en extrémité distale d'une tige dont la partie proximale est conçue pour être tenue à la main. Pour libérer l'autre main du chirurgien, il est alors nécessaire que l'actionnement en ouverture et fermeture de la micro-pince soit commandé par la main qui tient l'instrument. Il en résulte là encore un instrument complexe à fabriquer. Et pour qu'un tel instrument puisse être porté par le bras articulé d'un robot, ce dernier doit être suffisamment sophistiqué pour non seulement tenir l'instrument mais également actionner sa micro-pince en ouverture et fermeture.

Il peut ainsi être souhaité de prévoir un instrument de chirurgie otologique pour l'insertion d'un porte-électrodes d'implant cochléaire dans une oreille interne d'un patient qui permette de s'affranchir d'au moins une partie des problèmes et contraintes précités.

Il est donc proposé un instrument de ce type comportant une extrémité proximale de préhension et une extrémité distale fonctionnelle de capture et maintien d'une portion du porte-électrodes pendant l'insertion, dans lequel :
- l'extrémité proximale comporte des moyens de fixation à un bras articulé de robot ; et
- l'extrémité distale comporte un organe fixe de préhension par enserrement de la portion du porte-électrodes.

En présentant une extrémité proximale spécifiquement pourvue de moyens de fixation à un bras articulé de robot, il est possible de se libérer d'une contrainte en termes de disponibilité des mains du chirurgien et de prévoir une extrémité distale fonctionnelle de capture et maintien beaucoup plus simple que celles envisagées dans l'art antérieur. Ainsi, un organe fixe de préhension par enserrement, incluant en particulier le fait qu'il n'est pas articulé, est astucieusement proposé en combinaison avec les moyens de fixation à un bras de robot pour former un instrument simple et peu coûteux à fabriquer. Il nécessite une action manuelle du chirurgien pour capturer ou libérer la portion de porte-électrodes souhaitée puisqu'il est fixe et en particulier non articulé, mais cela n'est plus un problème compte tenu de son extrémité proximale spécifiquement prévue pour être tenue par un bras de robot.

De façon optionnelle, l'organe fixe de préhension par enserrement est en outre rigide, l'enserrement de la portion du porte-électrodes se faisant par élasticité de cette dernière.

Dans ce cas, l'instrument de chirurgie otologique peut être constitué d'acier inoxydable chirurgical, notamment de type 304L ou 316L selon la norme AISI.

De façon optionnelle également, l'organe fixe de préhension par enserrement est une fourche à deux dents s'étendant dans le prolongement d'une portion distale de l'instrument, pour une capture et un maintien de la portion de porte-électrodes entre ces deux dents.

Dans ce cas, l'instrument de chirurgie otologique peut comporter plusieurs portions déviées s'étendant toutes dans un même plan parallèle à un plan principal des deux dents de la fourche.

Dans ce cas également, l'instrument de chirurgie otologique peut comporter plusieurs portions déviées s'étendant toutes dans un même plan perpendiculaire à un plan principal des deux dents de la fourche.

De façon optionnelle également, chaque dent de la fourche est de section rectangulaire.

De façon optionnelle également, l'organe fixe de préhension par enserrement est une gouttière rapportée latéralement contre l'extrémité libre d'une portion distale de l'instrument, pour une capture et un maintien de la portion de porte-électrodes dans le volume intérieur de la gouttière.

De façon optionnelle également, les moyens de fixation au bras articulé de robot comportent une gorge de verrouillage creusée longitudinalement dans la face externe de l'extrémité proximale de l'instrument.

Il est également proposé une installation robotisée d'intervention chirurgicale comportant :
- un robot muni d'un bras articulé déplaçable sur commande électronique ; et
- un instrument de chirurgie otologique (30 ; 30') selon l'une quelconque des revendications 1 à 9 ;
dans laquelle le bras articulé du robot présente des moyens de fixation complémentaires aptes à coopérer avec les moyens de fixation de l'instrument de chirurgie otologique.

L'invention sera mieux comprise à l'aide de la description qui va suivre, donnée uniquement à titre d'exemple et faite en se référant aux dessins annexés dans lesquels :
[Fig.1] la figure 1 représente schématiquement la structure générale d'un exemple de partie interne d'un implant cochléaire avec son porte-électrodes,
[Fig.2] la figure 2 représente schématiquement et en vue longitudinale la structure générale d'un instrument de chirurgie otologique pour l'insertion d'un porte-électrodes tel que celui de la figure 1, selon un premier mode de réalisation de l'invention,
[Fig.3] la figure 3 illustre l'instrument de la figure 2 en perspective,
[Fig.4] la figure 4 illustre une variante de l'instrument de la figure 2 en perspective,
[Fig.5] la figure 5 représente schématiquement et en perspective la structure générale d'un instrument de chirurgie otologique pour l'insertion d'un porte-électrodes tel que celui de la figure 1, selon un deuxième mode de réalisation de l'invention,
[Fig.6] la figure 6 est un ensemble de deux vues de côté et de face d'une extrémité distale fonctionnelle de l'instrument de la figure 5,
[Fig.7] la figure 7 est un ensemble de deux vues de côté et de face d'une variante pour l'extrémité distale fonctionnelle de l'instrument de la figure 5,
[Fig.8] la figure 8 représente schématiquement et partiellement la structure générale d'une installation robotisée d'intervention chirurgicale incluant l'instrument de la figure 2, et
[Fig.9] la figure 9 illustre les étapes successives d'un procédé d'intervention chirurgicale à l'aide de l'installation robotisée de la figure 8.

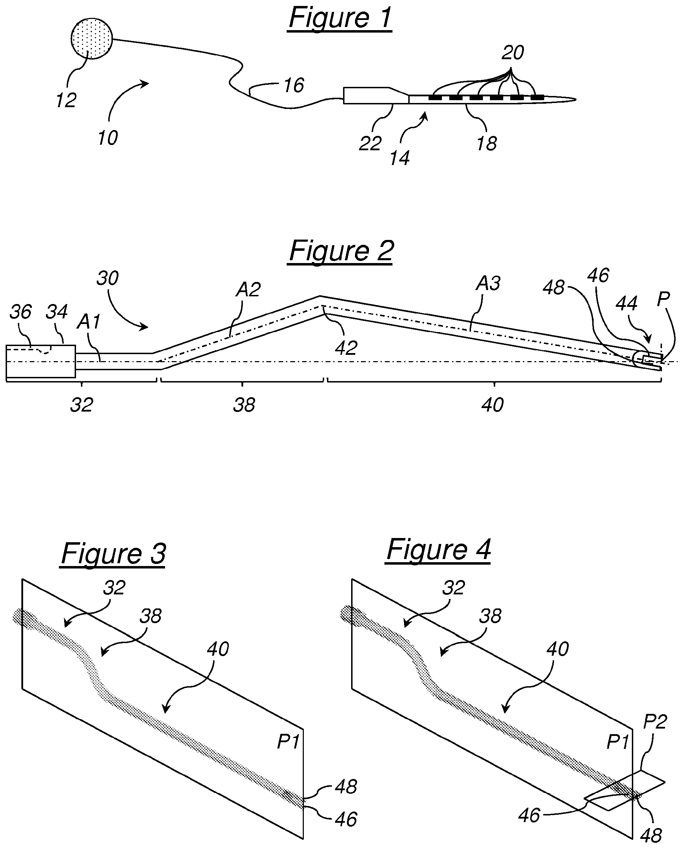

La partie interne 10 d'implant cochléaire illustrée sur la figure 1 comporte un récepteur 12 relié à un porte-électrodes 14 à l'aide d'un raccordement électrique 16. Le récepteur 12 est destiné à être placé sous la peau d'un individu en vis-à-vis du transmetteur de la partie externe non illustrée. Le porte-électrodes 14 est destiné à être placé dans la rampe tympanique de la cochlée de l'individu.

Différents types de porte-électrodes existent sur le marché. Celui présenté dans la figure 1 présente un corps principal 18 formant support pour une pluralité d'électrodes 20 ainsi qu'un aileron proximal 22 facilitant sa saisie. Le corps principal 18 est souple et élastique, généralement constitué de silicone, de forme essentiellement cylindrique à section circulaire. L'aileron proximal 22 est optionnel, souple, élastique et généralement constitué de silicone comme le corps principal 18, et prolonge ce dernier selon une section plate rectangulaire.

L'instrument de chirurgie otologique 30 représenté schématiquement sur la figure 2 est adapté pour l'insertion du porte-électrodes de la figure 1 dans l'oreille interne d'un patient en le saisissant par son aileron proximal 22. Conformément à un mode de réalisation avantageux mais optionnel, il présente plusieurs portions déviées permettant de libérer l'axe de visée du chirurgien. En variante, il pourrait être rectiligne.

Il comporte ainsi une première portion de maintien ou fixation 32, s'étendant autour d'un premier axe rectiligne A1 et présentant une extrémité proximale de préhension 34 autour de ce premier axe rectiligne A1. L'extrémité proximale de préhension 34 comporte des moyens 36 de fixation à un bras articulé de robot, par exemple matérialisés par une simple gorge de verrouillage de section semi-circulaire creusée longitudinalement dans sa face externe et se terminant par une cavité partiellement sphérique de plus grande profondeur en conformité avec l'enseignement du brevet FR 2 998 344 B1.

L'instrument de chirurgie otologique 30 comporte en outre une deuxième portion de déviation 38, solidaire de la première portion 32, s'étendant sans degré de liberté à partir de la première portion 32 dans la direction principale d'un deuxième axe rectiligne A2 l'éloignant du premier axe rectiligne A1. L'angle entre les axes A1 et A2 est par exemple compris entre 2 et 10 degrés, de préférence entre 2 et 4 degrés, pour donner une forme très allongée à l'instrument 30 avec un encombrement latéral minimal.

L'instrument de chirurgie otologique 30 comporte en outre une troisième portion de support fonctionnel 40, solidaire de la deuxième portion 38, s'étendant sans degré de liberté à partir d'une extrémité distale 42 de la deuxième portion 38 autour d'un troisième axe rectiligne A3 différent du deuxième axe rectiligne A2 et la rapprochant du premier axe rectiligne A1. Cette troisième portion 40 présente une extrémité distale fonctionnelle 44 qui, conformément à la présente invention, comporte un organe fixe de préhension par enserrement d'une portion souhaitée du porte-électrodes 14. L'angle entre les axes A1 et A3 est par exemple compris entre 2 et 10 degrés, de préférence entre 2 et 4 degrés. Il doit être suffisant pour dégager le champ de vision d'un utilisateur et faciliter le geste chirurgical, tout en limitant l'encombrement latéral de l'instrument chirurgical 30. De façon avantageuse mais optionnelle, conformément aux principes généraux de la demande de brevet FR 3 066 378 A1, la troisième portion 40 est conformée, par sa longueur et par l'orientation du troisième axe rectiligne A3, de telle sorte que le premier axe rectiligne A1 autour duquel s'étend la première portion 32 passe par l'extrémité distale fonctionnelle 44 - et plus précisément par l'organe fixe de préhension par enserrement - de la troisième portion 40 en un point pivot P.

Les trois portions 32, 38 et 40 de l'instrument de chirurgie otologique 30 peuvent être constituées, à l'exception éventuelle de l'extrémité proximale de préhension 34 et de l'extrémité distale fonctionnelle 44, d'une seule et même tige (simple tige cylindrique de section circulaire, carré ou rectangle, ou tringlerie) présentant deux coudées : la première coudée à la jonction des première et deuxième portions 32, 38 ; la deuxième coudée à la jonction des deuxième et troisième portions 38, 40, c'est-à-dire à l'extrémité distale 42 de la deuxième portion 38. Par ailleurs, tous les éléments constitutifs de l'instrument 30 décrits précédemment peuvent être conçus en plastique, métal, acier inoxydable, titane ou en d'autres matériaux rigides ou associations de matériaux rigides biocompatibles adaptés à une application chirurgicale en otorhinolaryngologie. Un exemple de matériau particulièrement recommandé en chirurgie otologique est l'acier inoxydable chirurgical de type 304L ou 316L selon la norme AISI (de l'anglais « American Iron and Steel Institute ») qui peut constituer l'ensemble de l'instrument. Dans ce cas, l'organe fixe de préhension par enserrement formant l'extrémité distale fonctionnelle 44 peut être venu de matière avec le reste de la tige formant les trois portions déviées 32, 38 et 40. L'intérêt d'avoir un organe fixe de préhension par enserrement rigide comme le reste de l'instrument est la simplicité de fabrication de l'ensemble et sa conformité aux exigences chirurgicales. L'enserrement de la portion souhaitée du porte-électrodes 14 peut de toute façon se faire grâce à l'élasticité de cette dernière.

Lorsque l'extrémité proximale de préhension 34 est une pièce rapportée sur la tige mentionnée ci-dessus, elle peut être constituée d'un manchon essentiellement cylindrique comprenant un alésage le long de l'axe A1 pour recevoir l'extrémité proximale de la tige. De manière similaire, lorsque l'extrémité distale fonctionnelle 44 est une pièce rapportée sur la tige mentionnée ci-dessus, elle peut comporter une saillie cylindrique destinée à être reçue dans un alésage le long de l'axe A3 en partie distale de la tige. L'intérêt d'une extrémité distale fonctionnelle 44 conçue en pièce rapportée est que le reste de l'instrument 30 peut être fabriqué de façon standard, en tant qu'instrument « universel », et fourni avec un ensemble d'extrémités distales amovibles à différentes fonctions chirurgicales autres que celle *a minima* de préhension par enserrement d'une portion du porte-électrodes 14.

En termes de dimensions et conformations, l'instrument 30 doit généralement être de longueur totale (entre l'extrémité proximale et le point pivot P) comprise entre 10 et 20 cm, de préférence entre 10 et 15 cm, pour une utilisation en chirurgie otologique. Par exemple, lorsque cette longueur est voisine de 14 cm avec un angle entre les axes A1 et A3 d'environ 3,2 degrés, cela permet de déporter la distance latérale d'observation de 1 cm, lorsque l'œil de l'utilisateur est à 18 cm du point pivot P, c'est-à-dire à environ 4 cm de distance longitudinale de l'extrémité proximale de l'instrument 30 le long de l'axe A1. Cette configuration s'avère satisfaisante à l'usage. Par ailleurs, un diamètre de tige cylindrique compris entre 1 et 2 mm convient également. On notera toutefois que ces valeurs sont données à titre indicatif et qu'elles pourraient en particulier être plus petites ou plus grandes en fonction des applications et contextes visés.

Selon le mode de réalisation de la figure 2, l'organe fixe de préhension par enserrement est plus précisément une fourche à deux dents 46 et 48 s'étendant dans le prolongement de la troisième portion 40 de l'instrument 30, pour une capture et un maintien de l'aileron proximal 22 du porte-électrodes 14 entre ces deux dents. La forme et les dimensions des deux dents 46, 48 sont à définir en fonction de la forme et des dimensions de l'aileron proximal 22 ou de toute autre portion du porte-électrodes 14 que l'on souhaite saisir et maintenir. En particulier, dans le cas d'un aileron proximal de section plate rectangulaire comme celui de la figure 1, des dents parallèles de sections rectangulaires espacées à un jeu négatif près en fonction de l'épaisseur de l'aileron sont appropriées. En variante, elles pourraient être de sections autres que rectangulaires, notamment rondes, ovales, etc.

La vue en perspective de la figure 3 montre que l'instrument de chirurgie otologique 30, incluant ses trois portions déviées 32, 38, 40, s'étend dans un plan P1 qui est parallèle au plan principal dans lequel s'étendent les deux dents 46 et 48. C'est même le même plan.

En variante, la vue en perspective de la figure 4 montre que l'instrument de chirurgie otologique 30, incluant ses trois portions déviées 32, 38, 40, s'étend dans un plan P1 qui peut être perpendiculaire au plan principal P2 dans lequel s'étendent parallèlement les deux dents 46 et 48. D'autres variantes sont également possibles et dépendent principalement de la forme du porte-électrodes à capturer et maintenir ainsi que de la façon dont on souhaite l'insérer dans l'oreille interne du patient.

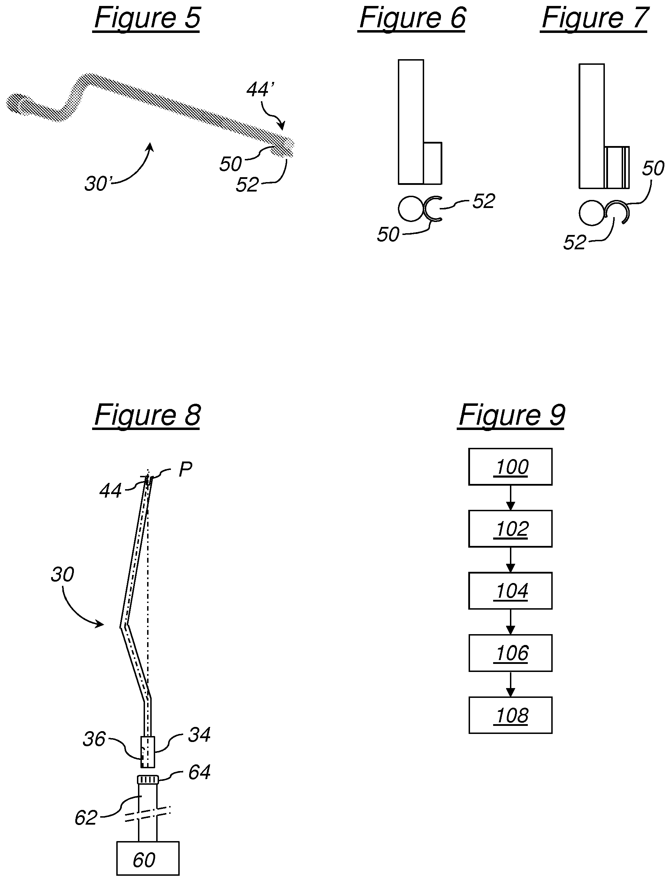

L'instrument de chirurgie otologique 30' illustré sur la figure 5 diffère de ceux des figures 2 à 4 par son extrémité distale fonctionnelle 44' dont l'organe fixe de préhension par enserrement n'est plus une fourche mais une gouttière 50, c'est-à-dire un élément de forme cylindrique creuse à ouverture longitudinale 52 sur toute sa longueur, cette gouttière 50 étant rapportée latéralement contre l'extrémité libre de la troisième portion rectiligne de l'instrument 30'. Cela permet une capture et un maintien du corps principal cylindrique 18 du porte-électrodes 14 dans le volume intérieur du cylindre creux et ouvert formé par la gouttière 50. C'est notamment un mode de réalisation préféré dans le cas où le porte-électrodes 14 ne comporte pas d'aileron proximal 22. L'ouverture longitudinale est suffisamment large pour capturer facilement le porte-électrodes 14 mais également suffisamment étroite pour le maintenir pendant son insertion dans l'oreille interne du patient. En d'autres termes, les bords de la gouttière 50 sont avantageusement repliés l'un vers l'autre pour former en section un arc de cercle légèrement supérieur au demi-cercle.

Les vues de côté et de face de la figure 6 montrent que l'ouverture longitudinale 52 est opposée à la troisième portion de tige contre laquelle est fixée la gouttière 50.

Les vues de côté et de face de la figure 7 montrent une variante selon laquelle l'ouverture longitudinale 52 est angulairement décalée d'environ 90 degrés pour être positionnée latéralement par rapport à la troisième portion de tige. D'autres variantes sont également possibles et dépendent principalement de la forme du porte-électrodes à capturer et maintenir ainsi que de la façon dont on souhaite l'insérer dans l'oreille interne du patient.

Une installation robotisée d'intervention chirurgicale exploitant l'instrument de chirurgie otologique 30 est illustrée de façon très schématique sur la figure 8. Elle comporte un robot 60 muni d'un bras porteur 62 déplaçable sur commande électronique. Un exemple de robot compatible avec une installation selon l'invention est donné dans l'article de Miroir et al, intitulé « RobOtol : from design to evaluation of a robot for middle ear surgery », publié à l'occasion de la conférence IEEE/RSJ International Conference on Intelligent Robots and Systems qui s'est tenue du 18 au 22 octobre 2010 à Taipei (TW). Il présente une architecture et une cinématique particulièrement bien adaptées aux interventions chirurgicales otologiques de l'oreille moyenne ou interne de patients, notamment celles concernant l'insertion de la partie interne d'un implant cochléaire.

L'installation illustrée sur la figure 8 comporte en outre :
- l'instrument de chirurgie otologique 30 dont l'extrémité proximale de préhension 34 est munie des moyens de fixation 36 à gorge de verrouillage, et
- des moyens complémentaires de fixation de l'extrémité proximale de préhension 34 de l'instrument 30 au bras porteur 62 du robot 60.

Ces moyens de fixation complémentaires comportent par exemple une bague de serrage 64 disposée à l'extrémité distale libre du bras porteur 62 du robot 60, ce bras porteur 62 étant lui-même destiné à recevoir par insertion l'extrémité proximale de préhension 34. La gorge de verrouillage 36 permet d'accompagner et guider angulairement l'insertion de l'instrument 30 dans le bras porteur 62 du robot 60, comme enseigné dans le brevet FR 2 998 344 B1. Plus généralement, tous moyens de fixation connus et compatibles avec la configuration de l'installation illustrée sur la figure 9 sont envisageables, notamment tous moyens permettant de fixer l'instrument de chirurgie otologique 30 de telle sorte que son axe principal A1 corresponde avec l'axe d'intervention du robot 60.

Bien évidemment, l'instrument 30 peut être remplacé par l'instrument 30' de la figure 5.

La figure 9 illustre les étapes successives d'un procédé d'intervention chirurgicale pour l'insertion par un chirurgien du porte-électrodes 14 dans la cochlée d'un patient à l'aide de l'installation robotisée de la figure 8.

Au cours d'une première étape 100, le chirurgien fixe l'instrument chirurgical otologique 30 ou 30' à l'extrémité libre du bras articulé 62 du robot 60.

Au cours d'une étape suivante 102, il saisit le porte-électrodes 14 et enserre la portion souhaitée, aileron proximal 22 ou corps principal 18, dans l'organe fixe de préhension 44, 46 ou 50.

Au cours d'une étape 104 d'insertion du porte-électrodes 14 dans l'oreille interne du patient, le chirurgien dirige précisément le porte-électrodes 14 vers l'intérieur de la cochlée en déplaçant l'extrémité distale fonctionnelle 44 ou 44' de l'instrument de chirurgie otologique 30 ou 30' porté par le bras articulé 62 du robot 60 à l'aide d'un périphérique de commande (non illustré). Le geste chirurgical est ainsi très précis et contrôlé.

Lorsque le porte-électrodes 14 est correctement installé dans la cochlée, le chirurgien libère manuellement sa portion enserrée dans l'organe fixe de préhension 44, 46 ou 50 au cours d'une étape 106.

Enfin, au cours d'une dernière étape 108, le chirurgien retire l'instrument de chirurgie otologique 30 ou 30' de la zone opératoire en commandant précisément son déplacement à l'aide du périphérique de commande.

Il apparaît clairement qu'une installation robotisée telle que celle décrite précédemment permet de faciliter et sécuriser une intervention chirurgicale d'insertion d'un porte-électrodes d'implant cochléaire dans l'oreille interne d'un patient à l'aide d'un instrument simple à concevoir, fabriquer et utiliser. La gestuelle connue peut être reproduite en éliminant les tremblements, les mouvements involontaires et en assurant une reproductibilité du geste chirurgical.

L'invention est définie par les revendications suivantes.

## Revendications

1. Instrument de chirurgie otologique (30 ; 30') pour l'insertion d'un porte-électrodes (14) d'implant cochléaire (10) dans une oreille interne d'un patient, comportant une extrémité proximale de préhension (34) et une extrémité distale fonctionnelle (44 ; 44') de capture et maintien d'une portion (22 ; 18) du porte-électrodes (14) pendant l'insertion, tel que :
- l'extrémité proximale (34) comporte des moyens (36) de fixation à un bras articulé (62) de robot (60) ; et
- l'extrémité distale (44 ; 44') comporte un organe (46, 48 ; 50) de préhension par enserrement de la portion (22 ; 18) du porte-électrodes (14) ;
**caractérisé en ce que** l'organe de préhension par enserrement est fixe, incluant en particulier le fait qu'il n'est pas articulé.

2. Instrument de chirurgie otologique (30 ; 30') selon la revendication 1, dans lequel l'organe fixe (46, 48 ; 50) de préhension par enserrement est en outre rigide, l'enserrement de la portion (22 ; 18) du porte-électrodes (14) se faisant par élasticité de cette dernière.

3. Instrument de chirurgie otologique (30 ; 30') selon la revendication 2, constitué d'acier inoxydable chirurgical, notamment de type 304L ou 316L selon la norme AISI.

4. Instrument de chirurgie otologique (30) selon l'une quelconque des revendications 1 à 3, dans lequel l'organe fixe de préhension par enserrement est une fourche à deux dents (46, 48) s'étendant dans le prolongement d'une portion distale (40) de l'instrument (30), pour une capture et un maintien de la portion (22) de porte-électrodes (14) entre ces deux dents (46, 48).

5. Instrument de chirurgie otologique (30) selon la revendication 4, comportant plusieurs portions déviées (32, 38, 40) s'étendant toutes dans un même plan (P1) parallèle à un plan principal (P1) des deux dents (46, 48) de la fourche.

6. Instrument de chirurgie otologique (30) selon la revendication 4, comportant plusieurs portions déviées (32, 38, 40) s'étendant toutes dans un même plan (P1) perpendiculaire à un plan principal (P2) des deux dents (46, 48) de la fourche.

7. Instrument de chirurgie otologique (30) selon l'une quelconque des revendications 4 à 6, dans lequel chaque dent (46, 48) de la fourche est de section rectangulaire.

8. Instrument de chirurgie otologique (30') selon l'une quelconque des revendications 1 à 3, dans lequel l'organe fixe de préhension par enserrement est une gouttière (50) rapportée latéralement contre l'extrémité libre (44') d'une portion distale (40) de l'instrument (30'), pour une capture et un maintien de la portion (18) de porte-électrodes (14) dans le volume intérieur de la gouttière (50).

9. Instrument de chirurgie otologique (30 ; 30') selon l'une quelconque des revendications 1 à 8, dans lequel les moyens (36) de fixation au bras articulé (62) de robot (60) comportent une gorge de verrouillage creusée longitudinalement dans la face externe de son extrémité proximale (34).

10. Installation robotisée d'intervention chirurgicale comportant :
- un robot (60) muni d'un bras articulé (62) déplaçable sur commande électronique ; et
- un instrument de chirurgie otologique (30 ; 30') selon l'une quelconque des revendications 1 à 9 ;
dans laquelle le bras articulé (62) du robot (60) présente des moyens de fixation complémentaires (64) aptes à coopérer avec les moyens de fixation (36) de l'instrument de chirurgie otologique (30 ; 30').

## Patentansprüche

1. Ohrenchirurgie-Instrument (30; 30') zum Einführen eines Elektrodenhalters (14) eines Cochlea-Implantats (10) in ein Innenohr eines Patienten, das ein proximales Greifende (34) und ein funktionelles distales Ende (44; 44') zum Erfassen und Halten eines Abschnitts (22; 18) des Elektrodenhalters (14) während des Einführens umfasst, so beschaffen, dass:
- das proximale Ende (34) Mittel (36) zur Befestigung an einem Gelenkarm (62) eines Roboters (60) umfasst; und
- das distale Ende (44; 44') ein Organ (46, 48; 50) zum Greifen des Abschnitts (22; 18) des Elektrodenhalters (14) durch Einspannen umfasst;
**dadurch gekennzeichnet, dass** das Einspann-Greiforgan feststehend ist, insbesondere einschließlich der Tatsache, dass es nicht gelenkig ist.

2. Ohrenchirurgie-Instrument (30; 30') nach Anspruch 1, wobei das feststehende Einspann-Greiforgan (46, 48; 50) ferner starr ist, wobei das Einspannen des Abschnitts (22; 18) des Elektrodenhalters (14) durch dessen Elastizität erfolgt.

3. Ohrenchirurgie-Instrument (30; 30') nach Anspruch 2, das aus chirurgischem Edelstahl besteht, insbesondere vom Typ 304L oder 316L gemäß dem AISI-Standard.

4. Ohrenchirurgie-Instrument (30) nach einem der Ansprüche 1 bis 3, wobei das feststehende Einspann-Greiforgan eine Gabel mit zwei Zinken (46, 48) ist, die sich in der Verlängerung eines distalen Abschnitts (40) des Instruments (30) erstrecken, um den Abschnitt (22) des Elektrodenhalters (14) zwischen diesen beiden Zinken (46, 48) zu erfassen und zu halten.

5. Ohrenchirurgie-Instrument (30) nach Anspruch 4, das mehrere abgewinkelte Abschnitte (32, 38, 40) umfasst, die sich alle in einer gleichen Ebene (P1) parallel zu einer Hauptebene (P1) der beiden Zinken (46, 48) der Gabel erstrecken.

6. Ohrenchirurgie-Instrument (30) nach Anspruch 4, das mehrere abgewinkelte Abschnitte (32, 38, 40) umfasst, die sich alle in einer gleichen Ebene (P1) senkrecht zu einer Hauptebene (P2) der beiden Zinken (46, 48) der Gabel erstrecken.

7. Ohrenchirurgie-Instrument (30) nach einem der Ansprüche 4 bis 6, wobei jeder Zinken (46, 48) der Gabel einen rechteckigen Querschnitt hat.

8. Ohrenchirurgie-Instrument (30') nach einem der Ansprüche 1 bis 3, wobei das feststehende Einspann-Greiforgan eine Schiene (50) ist, die seitlich an dem freien Ende (44') eines distalen Abschnitts (40) des Instruments (30') angebracht ist, um den Abschnitt (18) des Elektrodenhalters (14) im Innenvolumen der Schiene (50) zu erfassen und zu halten.

9. Ohrenchirurgie-Instrument (30; 30') nach einem der Ansprüche 1 bis 8, wobei die Befestigungsmittel (36) an dem Gelenkarm (62) des Roboters (60) eine Verriegelungsnut umfassen, die längs vertieft in der Außenfläche seines proximalen Endes (34) ausgebildet ist.

10. Robotergestützte Vorrichtung für chirurgische Eingriffe, umfassend:
- einen Roboter (60), der mit einem Gelenkarm (62) ausgestattet ist, der mittels elektronischer Steuerung bewegt werden kann; und
- ein Ohrenchirurgie-Instrument (30; 30') nach einem der Ansprüche 1 bis 9;
wobei der Gelenkarm (62) des Roboters (60) ergänzende Befestigungsmittel (64) aufweist, die dazu ausgelegt sind, mit den Befestigungsmitteln (36) des Ohrenchirurgie-Instruments (30; 30') zusammenzuwirken.

## Claims

1. An otologic surgery instrument (30; 30') for inserting a cochlear implant (10) electrode holder (14) into a patient's inner ear, comprising a gripping proximal end (34) and a functional distal end (44; 44') for capturing and holding a portion (22; 18) of the electrode holder (14) during insertion, wherein:
- the proximal end (34) comprises means (36) for fastening to an articulated arm (62) of a robot (60); and
- the distal end (44; 44') comprises a member (46, 48; 50) for gripping the portion (22; 18) of the electrode holder (14) by clamping;
**characterized in that** the member for gripping by clamping is fixed, including in particular the fact **in that** it is not articulated.

2. The otologic surgery instrument (30; 30') as claimed in claim 1, wherein the fixed gripping member (46, 48; 50) is furthermore rigid, the gripping of the portion (22; 18) of the electrode holder (14) being done by elasticity of the latter.

3. The otologic surgery instrument (30; 30') as claimed in claim 2, made of surgical stainless steel, in particular of 304L or 316L type according to AISI standard.

4. The otologic surgery instrument (30) as claimed in any one of claims 1 to 3, wherein the fixed gripping member by clamping is a two-pronged fork (46, 48) extending along a distal portion (40) of the instrument (30), for capturing and holding the portion (22) of electrode holder (14) between these two prongs (46, 48).

5. The otologic surgery instrument (30) as claimed in claim 4, comprising several deviated portions (32, 38, 40) all extending in one and the same plane (P1) parallel to a main plane (P1) of the two prongs (46, 48) of the fork.

6. The otologic surgery instrument (30) as claimed in claim 4, comprising several deviated portions (32, 38, 40) all extending in one and the same plane (P1) perpendicular to a main plane (P2) of the two prongs (46, 48) of the fork.

7. The otologic surgery instrument (30) as claimed in any one of claims 4 to 6, wherein each prong (46, 48) of the fork has a rectangular cross section.

8. The otologic surgery instrument (30') as claimed in any one of claims 1 to 3, wherein the fixed gripping member by clamping is a gutter (50) attached laterally to the free end (44') of a distal portion (40) of the instrument (30'), for capturing and holding the portion (18) of electrode holder (14) within the interior volume of the gutter (50).

9. The otologic surgery instrument (30; 30') as claimed in any one of claims 1 to 8, wherein the means (36) for fastening to the articulated arm (62) of the robot (60) comprise a locking groove hollowed longitudinally in the outer face of its proximal end (34).

10. A robotic surgical intervention installation comprising:
- a robot (60) with an articulated arm (62) that can be moved under electronic control; and
- an otologic surgery instrument (30; 30') as claimed in any of claims 1 to 9;
wherein the articulated arm (62) of the robot (60) has complementary fastening means (64) adapted to cooperate with the fastening means (36) of the otologic surgery instrument (30; 30'
